# EUROPEAN PATENT SPECIFICATION

(11) **EP 1 602 390 B1**
(45) Date of publication and mention of the grant of the patent: **09.10.2013**
(21) Application number: 05253399.9
(22) Date of filing: 02.06.2005
(51) Int. Cl.: A61M 16/08

(54) **Improvements relating to medical respiratory apparatus**
Verbesserungen an einen medizinischen Atemgerät
Améliorations aux appareils respiratoires médicaux

(30) Priority: 03.06.2004 GB 0412482
(43) Date of publication of application: 07.12.2005
(73) Proprietor: Intersurgical AG, Vaduz (LI)
(72) Inventor: Payne, Simon Robert, Dunsfold, Surrey GU8 4LU (GB)
(74) Representative: Adamson Jones

(56) References cited:
- EP-A- 0 595 499
- EP-A2- 0 150 912
- DE-U1- 29 508 911
- GB-A- 2 199 630
- US-A- 4 637 384
- US-A- 5 746 199

## Description

This invention relates to improvements in medical respiratory apparatus, in particular medical respiratory apparatus comprising a number of cooperating components forming a fluid conduit through which inhaled and/or exhaled gases flow, and having at least one port allowing insertion of an ancillary device into that fluid conduit.

Medical respiratory apparatus typically includes a number of cooperating components that each have a fluid conduit terminating at each end with a male/female end portion that is engageable with a male/female end portion of a fluid conduit of an adjacent component. In this way, the fluid conduits of adjacent components communicate with one another to form a combined fluid conduit through which inhaled and/or exhaled gases flow.

There are many different applications of medical respiratory apparatus which require the insertion of ancillary devices into the fluid conduit of the apparatus. Such applications include pressure monitoring, temperature monitoring, suctioning, gas sampling and fibre optic bronchoscopy. Typical medical respiratory apparatus therefore includes at least one port allowing access to the fluid conduit. These access ports are typically sealed when not in use by suitable closures.

A common arrangement comprises a first component, such as a swivel elbow connector, having a first fluid conduit and an access port that allows insertion of an ancillary device into the first fluid conduit, and a second component, such as an endotracheal tube device, having a second fluid conduit. Conventionally, the first fluid conduit has a female end portion adapted to closely receive a corresponding male end portion of the second fluid conduit with an interference fit so that the first and second fluid conduits are in communication with one another. The end of the male end portion situated within the female end portion thereby forms a circumferential shoulder on the interior surface of the combined fluid conduit.

Ancillary components inserted into, and passed along, a combined fluid conduit that extends through a plurality of cooperating components are therefore liable to catch, or even become obstructed by, such shoulders formed at the junctions between adjacent components. This may damage the device and may increase the time taken to complete the insertion procedure, hence increasing discomfort for the patient.

US 5,746,199, GB 2199630 and DE 29508911 each disclose a component of respiratory apparatus having a formation that acts to guide an ancillary device, such as a suction catheter, through an opening or fluid conduit, the components being specifically adapted to prevent the flow of respiratory gases through the guide formation. EP 0595499 discloses suction catheter assemblies that include a sealing device and a one-way valve. The sealing devices each have a central aperture through which the catheter is guided, with a sealed fit, and the one-way valve enables gas trapped inside the sleeve of the suction catheter to vent into the respiratory circuit when the gas pressure within the sleeve exceeds the gas pressure within the respiratory circuit. An example of a guide which allows flow of respiratory gases is disclosed in US4637384.

There has now been devised an improved medical respiratory apparatus and a component thereof which overcome or substantially mitigate the above-mentioned and/or other disadvantages associated with the prior art.

According to the invention, there is provided a first component of a medical respiratory apparatus, the first component comprising a female end portion adapted to closely receive a corresponding male end portion of a second component of the medical respiratory apparatus so that the first and second components are in communication with one another, the first component being further provided with an access port enabling insertion of an ancillary device, wherein the first component includes a guide formation formed with an inwardly facing deflecting surface which is arranged to deflect the ancillary device inwardly away from the interior surface of the female end portion, in use upon insertion of the ancillary device, thereby guiding the ancillary device into the second component, and preventing passage of the ancillary device into a peripheral portion of the female end portion of the first component in which the ancillary device would be liable to catch, or become obstructed by, a shoulder formed by the male end portion of the second component, characterised in that the guide formation includes one or more flow openings arranged to permit airflow through the guide formation without significant resistance such that inhaled and exhaled gases are able to flow in use, but through which the ancillary device is not able to pass.

According to a further aspect of the invention, there is provided medical respiratory apparatus comprising a first component as described above and a second component, the female end portion of the first component being adapted to closely receive a corresponding male end portion of the second component so that the first and second components are in communication with one another, the access port of the first component enabling insertion of an ancillary device, wherein the guide formation of the first component guides the ancillary device into the second component during use.

The medical respiratory apparatus according to the invention is advantageous principally because the guide formation that guides the ancillary device into the second component may prevent the ancillary device catching, or becoming obstructed by, the shoulder formed by the end of the male end portion of the second component. Consequently, the ancillary device is less likely to be damaged during use, and the time taken to complete the insertion procedure, and hence the discomfort experienced by the patient, may be reduced significantly.

The medical respiratory apparatus according to the invention is preferably adapted for supplying gases to a patient and/or receiving gases from a patient. The medical respiratory apparatus preferably comprises a plurality of components, such as respiratory devices (eg nebulisers, humidifiers, etc), respiratory tubing, and connectors for such tubing, that include cooperating fluid conduits for respiratory gases.

The female end portion of the first component preferably forms part of a connector for connecting a fluid conduit of a respiratory device or respiratory tubing with a fluid conduit of a corresponding respiratory device or respiratory tubing. The first component is preferably, therefore, either a connector for a respiratory device or respiratory tubing, or a respiratory device or respiratory tubing having an integral connector. Most preferably, however, the first component is an elbow connector, which may be of a fixed or swivel type, as discussed in more detail below.

Similarly, the male end portion of the second component preferably forms part of a connector for connecting a fluid conduit of a respiratory device or respiratory tubing with a fluid conduit of a corresponding respiratory device or respiratory tubing. The first component is preferably, therefore, either a connector for a respiratory device or respiratory tubing, or a respiratory device or respiratory tubing having a integral connector. Most preferably, however, the second component is a device for delivering gases to a patient and/or receiving gases from a patient, or is a connector for such a device. In particular, the second component may be an endotracheal tube, or a connector for an endotracheal tube.

The guide formation preferably, therefore, defines an opening through which the ancillary device may pass into the second component.

The female end portion of the first component, and the male end portion of the second component, both preferably have a circular cross-sectional shape.

The opening through which the ancillary device may pass into the second component is preferably located centrally relative to the longitudinal axis of the male end portion of the second component. The opening is preferably circular in shape, and is preferably dimensioned to be only slightly larger than the cross-sectional area of the ancillary device with which the medical respiratory apparatus is to be used.

Most preferably, the guide formation comprises a plurality of webs that extend inwardly from an interior surface of the first component, and have inner ends that define an opening through which the ancillary device may pass. The webs preferably extend both inwardly and axially along the female end portion of the first component towards the male end portion of the second component so that the inwardly facing surface of the guide formation is inclined relative to the interior surface of the female end portion of the first component. The guide formation is therefore preferably generally frusto-conical in shape, and acts as a funnel for the ancillary device. A guide ring preferably connects the inner ends of the webs so as to define the opening through which the ancillary device may pass.

The webs of the guide formation may be arcuate in form so as to aid passage of the ancillary device along the inwardly facing surface of the guide formation. In particular, a portion of the inwardly facing surface adjacent to the guide ring or opening defined by the guide formation may be convex in form, and a portion of the inwardly facing surface adjacent to the interior surface of the first component may be concave in form.

The plurality of webs are preferably spaced apart so that flow openings are defined between adjacent webs. Each flow opening preferably has a maximum width that is sufficiently small to prevent the ancillary device passing therethrough. Preferably, therefore, each flow opening has a maximum width that is less than the diameter of the smallest ancillary device with which the apparatus is likely to be used. Most preferably, each flow opening has a maximum width of less than 1.2mm, which is the diameter of a conventional suction catheter suitable for use with neonatals. The webs are also preferably tapered so that the flow openings are generally rectangular in shape.

The provision of flow openings, as described above, enables the opening through which the ancillary device may pass to have a much smaller diameter than is possible with a guide formation that does not include flow openings. The opening may therefore be formed so that the largest ancillary device with which the apparatus is likely to be used passes through the opening with a relatively close fit. For example, the largest conventional suction catheter that might be used with the apparatus is likely to be one having a diameter of 6.9mm. The opening preferably, therefore, has a diameter of less than 10mm, more preferably less than 7.5mm, and most preferably about 7mm.

Clearly, therefore, the sizes of the flow openings and the opening through which the ancillary device may pass are selected according to the size of the ancillary devices with which the apparatus is likely to be used.

The access port preferably comprises an opening in a wall of the first component that faces the female end portion of the first component. Most preferably, the access port is sealed when not in use by a suitable closure. Most preferably, the first component has the general form of an elbow connector in which the first component has a main fluid conduit with the female end portion at one end and the access port at the other end, and a branch fluid conduit that extends from an intermediate portion of the main fluid conduit and has a male end portion at its other end. The female end portion and/or the male end portion of the elbow connector may be fixed relative to the remainder of the connector, such that the elbow connector is of the fixed type. Alternatively, the female end portion and/or the male end portion of the elbow connector may be rotatable relative to a main body of the connector, such that the elbow connector is of the swivel type.

It will be appreciated that, in addition to acting as a guide for an ancillary device, the guide formation may act as a barrier to the passage of foreign objects through the medical respiratory apparatus.

The invention will now be described in greater detail, by way of illustration only, with reference to the accompanying drawings, in which
Figure 1 is a side elevation, partly in section, of conventional medical respiratory apparatus during insertion of a suction catheter;
Figure 2 is a side elevation, partly in section, of medical respiratory apparatus similar to that of Figure 1, but modified in accordance with a first embodiment of the invention, during insertion of a suction catheter;
Figure 3 is an end elevation of parts of the medical respiratory apparatus of Figure 2; and
Figure 4 is a cross-sectional view of a connector that forms part of the medical respiratory apparatus shown in Figures 2 and 3.

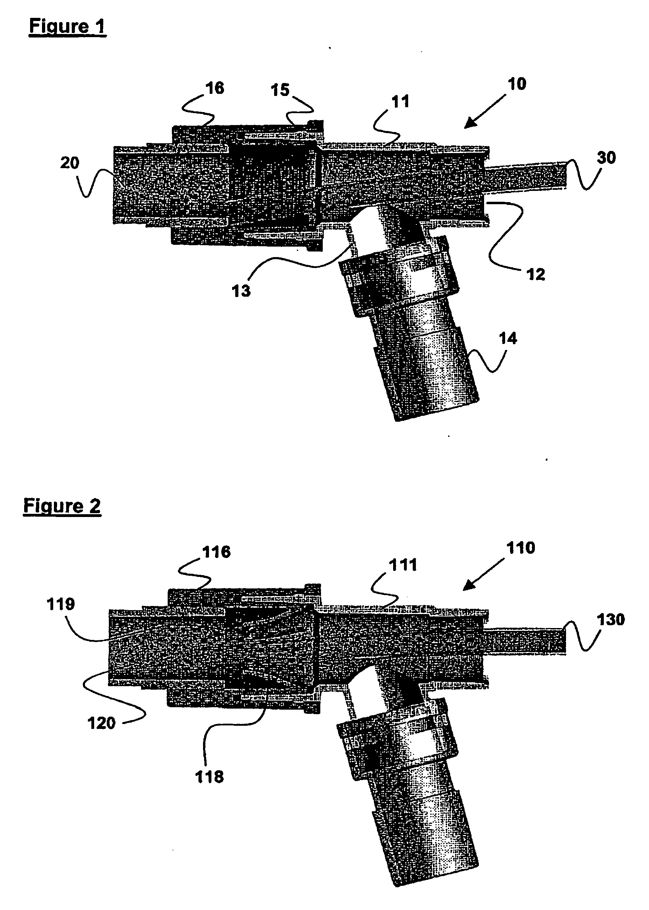

Figure 1 shows elements of conventional medical respiratory apparatus. These elements are a swivel elbow connector 10 engaged, via a first connector 16, with an endotracheal tube connector 20. A second connector 14 is attached to a branch limb 13 of the swivel elbow connector 10. Each of these components 10,14,16,20 is formed by injection moulding of a suitable plastics material. A suction catheter 30 is shown being inserted into the swivel elbow connector 10.

The swivel elbow connector 10 comprises a main fluid conduit 11 and the branch limb 13, which are both generally cylindrical in form and in fluid communication with one another. The main fluid conduit 11 has a proximal end portion defining an access port 12 through which the suction catheter 30 is inserted during use, and a distal end portion that is engaged with the first connector 16. The distal end portion of the main fluid conduit 11 is of increased diameter, and has a circumferential ridge 15 about its exterior surface.

The first connector 16 has the general form of an open-ended cylinder with an internal bore forming a fluid conduit. A proximal end portion of the first connector 16 is engaged with the main fluid conduit 11 of the swivel elbow connector 10, and a distal end portion of the first connector 16 is engaged with the endotracheal tube connector 20. The proximal end portion of the first connector 16 is of double-walled construction, so that an axially extending cylindrical recess is formed in that end portion. The internal surface of the outer wall of the recess is formed with four inwardly extending projections (not visible in Figure 1) that are equiangularly spaced around the mouth of the recess. Finally, the internal bore of the first connector 16 is tapered, its internal diameter increasing from the proximal end to the distal end.

The distal end portion of the main fluid conduit 11 is received within the cylindrical recess of the first connector 16 such that the circumferential ridge 15 of the distal end portion is engaged with a snap fit behind the four projections of the first connector 16. The engagement between the swivel elbow connector 10 and first connector 16 is such that these components may be rotated relative to one another about their shared longitudinal axis.

The endotracheal tube connector 20 has the general form of an open-ended cylinder with an internal bore forming a fluid conduit. An endotracheal tube (not shown in the Figures) fits securely about the external surface of a distal end portion of the endotracheal tube connector 20. A proximal end portion of the endotracheal tube connector 20 has a tapered external surface that is closely received within the internal bore of the first connector 16 with an interference fit. The tapered nature of the external surface of the proximal end of the endotracheal tube connector 20, and the internal bore of the first connector 16, ensures a secure interference fit between these components.

The medical apparatus shown in Figure 1 is entirely conventional. A major problem associated with such medical respiratory apparatus is clearly illustrated by Figure 1. In particular, the end of the endotracheal tube connector 20 situated within the first connector 16 forms a circumferential shoulder. As a consequence, the suction catheter 30 is likely to catch, or be obstructed by, the end of the endotracheal tube connector 20 on insertion into the endotracheal tube. The present invention provides a solution to this problem.

Figure 2 shows elements of medical respiratory apparatus corresponding to those of Figure 1, but modified in accordance with a first embodiment of the invention. The apparatus shown in Figure 2 comprises a swivel elbow connector 110 engaged, via a connector 116, with a conventional endotracheal tube connector 120. The swivel elbow connector 110 and the endotracheal tube connector 120 are identical to the corresponding components of Figure 1. Each of these components 110,120 is formed by injection moulding of a suitable plastics material. A conventional suction catheter 130 is shown being inserted into the swivel elbow connector 110.

The apparatus according to a first embodiment of the invention differs from the conventional apparatus of Figure 1 by the provision of a guide formation 118 on the interior surface of the connector 116. As shown in Figure 2, the guide formation 118 guides the suction catheter 130 through a circular opening 119 of reduced diameter relative to the diameter of the internal bore of the endotracheal tube connector 120. In this way, the suction catheter is guided into the endotracheal tube connector 120, and hence the endotracheal tube, without the suction catheter 130 catching, or being obstructed by, the end of the endotracheal tube connector 120.

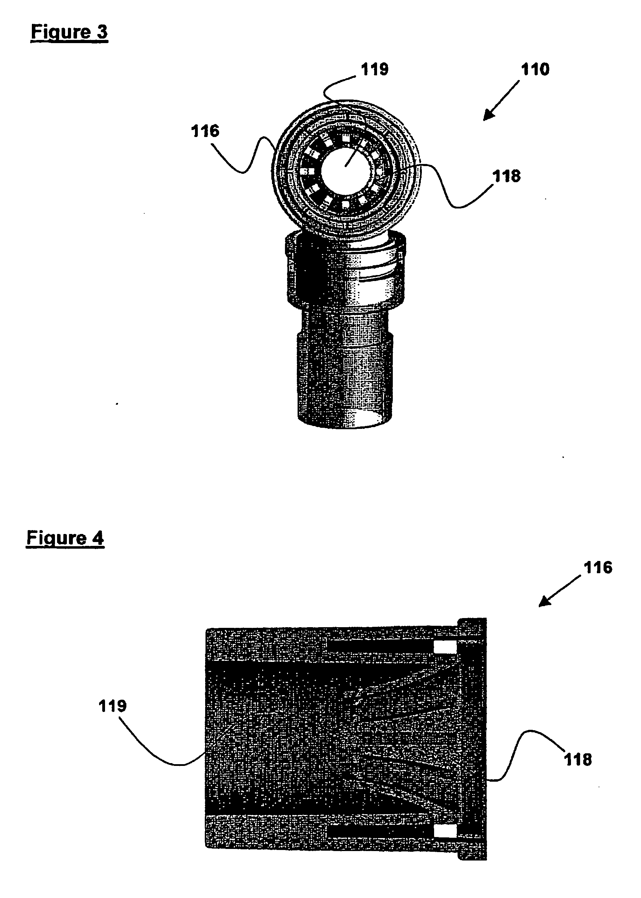

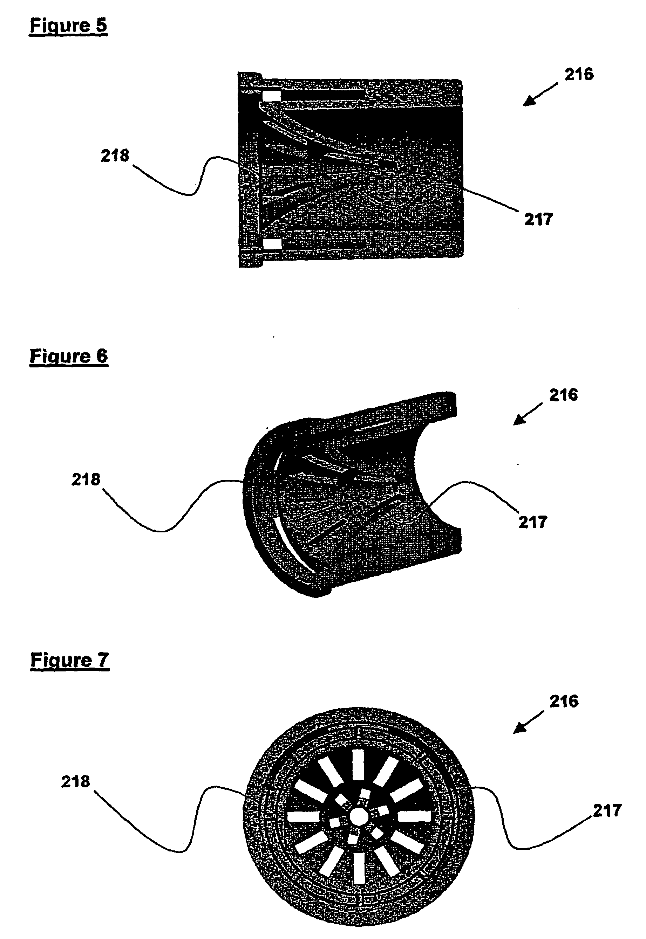

The guide formation 118 is shown more clearly in Figure 3 and Figure 4. The guide formation 118 is formed integrally with the connector 116 and comprises a plurality of webs that extend from the proximal end of the interior surface of the connector 116 to a guide ring of reduced diameter. The webs are spaced apart circumferentially and are inclined inwardly and axially towards the distal end of the connector 116, and hence the endotracheal tube connector 120. The guide formation 118 therefore acts as a funnel for the suction catheter 130. The webs are generally arcuate in form, having a convex inwardly facing surface.

The webs of the guide formation 118 are tapered in form so that the openings formed between adjacent webs are generally rectangular in shape. The openings formed between the webs of the guide formation 118 ensure that the resistance to air flow through the apparatus is not significantly increased by the presence of the guide formation 118.

The guide formation 118 of the swivel elbow connector 110 extends towards the endotracheal tube connector 120, but the opening 119 is spaced a short distance from the end of the endotracheal tube connector 120. Such an arrangement is suitable for ancillary devices (such as the suction catheter 130) that are sufficiently rigid to avoid the shoulder formed by the end of the endotracheal tube connector 120. However, to permit the use of particularly flexible ancillary devices, the guide formation 118 of the swivel elbow connector 110 may extend into the endotracheal tube connector 120.

## Claims

1. A first component (116) of a medical respiratory apparatus, the first component (116) comprising a female end portion adapted to closely receive a corresponding male end portion of a second component (120) of the medical respiratory apparatus so that the first and second components (116,120) are in communication with one another, the first component (116) being further provided with an access port enabling insertion of an ancillary device (130), wherein the first component (116) includes a guide formation (118) formed with an inwardly facing deflecting surface which is arranged to deflect the ancillary device inwardly away from the interior surface of the female end portion, in use upon insertion of the ancillary device, thereby guiding the ancillary device (130) into the second component (120), and preventing passage of the ancillary device into a peripheral portion of the female end portion of the first component in which the ancillary device would be liable to catch, or become obstructed by, a shoulder formed by the male end portion of the second component,
**characterised in that** the guide formation includes one or more flow openings arranged to permit airflow through the guide formation without significant resistance such that inhaled and exhaled gases are able to flow in use, but through which the ancillary device is not able to pass.

2. A first component (116) as claimed in Claim 1, wherein the guide formation (118) comprises a plurality of webs that extend inwardly from an interior surface of the first component (116), and have inner ends that define an opening (119) through which the ancillary device (130) may pass.

3. A first component (116) as claimed in Claim 2, wherein the webs extend both inwardly and axially along the female end portion of the first component (116) towards the male end portion of the second component (120) so that the inwardly facing surface of the guide formation (118) is inclined relative to the interior surface of the female end portion of the first component (116).

4. A first component (116) as claimed in Claim 3, wherein the guide formation (118) is generally frusto-conical in shape, and acts as a funnel for the ancillary device (130).

5. A first component (116) as claimed in any one of Claims 2 to 4, wherein the webs of the guide formation (118) are arcuate in form so as to aid passage of the ancillary device (130) along the inwardly facing surface of the guide formation (118).

6. A first component (116) as claimed in Claim 5, wherein a portion of the inwardly facing surface adjacent to the opening (119) defined by the guide formation (118) is convex in form, and a portion of the inwardly facing surface adjacent to the interior surface of the first component (116) is concave in form.

7. A first component (116) as claimed in any one of Claims 2 to 6, wherein the plurality of webs are spaced apart so that flow openings are defined between adjacent webs.

8. A first component (116) as claimed in any preceding claim, wherein the first component is either a connector for a respiratory device or respiratory tubing, or a respiratory device or respiratory tubing having an integral connector.

9. Medical respiratory apparatus comprising a first component (116) as claimed in any preceding claim and a second component (120), the female end portion of the first component (116) being adapted to closely receive a corresponding male end portion of the second component (120) so that the first and second components (116,120) are in communication with one another, and the access port of the first component (116) enabling insertion of an ancillary device (130),
wherein the guide formation (118) of the first embodiment (116) guides the ancillary device (130) into the second component (120) during use.

10. Medical respiratory apparatus as claimed in Claim 9, wherein the opening (119) through which the ancillary device (130) may pass into the second component (120) is located centrally relative to the longitudinal axis of the male end portion of the second component (120).

11. Medical respiratory apparatus as claimed in Claim 9 or Claim 10, wherein the medical respiratory apparatus is adapted for supplying gases to a patient and/or receiving gases from a patient.

## Patentansprüche

1. Erste Komponente (116) einer medizinischen Beatmungsvorrichtung, wobei die erste Komponente (116) einen weiblichen Endabschnitt aufweist, der so gestaltet ist, dass er einen entsprechenden männlichen Endabschnitt einer zweiten Komponente (120) der medizinischen Beatmungsvorrichtung eng aufnimmt, so dass die erste und die zweite Komponente (116, 120) in Verbindung miteinander sind, wobei die erste Komponente (116) ferner mit einem Zugangsanschluss versehen ist, der das Einführen einer Zusatzvorrichtung (130) ermöglicht, wobei die erste Komponente (116) eine Führungsformation (118) aufweist, die mit einer einwärts weisenden Ablenkfläche ausgebildet ist, die so ausgelegt ist, dass sie die Zusatzvorrichtung beim Gebrauch nach dem Einführen der Zusatzvorrichtung von der Innenfläche des weiblichen Endabschnitts weg ablenkt und dadurch die Zusatzvorrichtung (130) in die zweite Komponente (120) führt und die Passage der Zusatzvorrichtung in einen peripheren Abschnitt des weiblichen Endabschnitts der ersten Komponente verhütet, in der die Zusatzvorrichtung dazu neigt, an einen von dem männlichen Endabschnitt der zweiten Komponente gebildeten Ansatz anzuschlagen oder davon behindert zu werden, **dadurch gekennzeichnet, dass** die Führungsformation eine oder mehrere Strömungsöffnungen aufweist, die so ausgelegt sind, dass sie einen Luftstrom durch die Führungsformation ohne erheblichen Widerstand zulassen, so dass ein- und ausgeatmete Gase beim Gebrauch strömen können, sie aber die Zusatzvorrichtung nicht durchlassen.

2. Erste Komponente (116) nach Anspruch 1, wobei die Führungsformation (118) mehrere Stege umfasst, die von einer Innenfläche der ersten Komponente (116) einwärts verlaufen und innere Enden aufweisen, die eine Öffnung (119) definieren, durch die die Zusatzvorrichtung (130) passieren kann.

3. Erste Komponente (116) nach Anspruch 2, wobei die Stege sowohl einwärts als auch axial entlang dem weiblichen Endabschnitt der ersten Komponente (116) in Richtung auf den männlichen Endabschnitt der zweiten Komponente (120) verlaufen, so dass die einwärts weisende Fläche der Führungsformation (118) relativ zur Innenfläche des weiblichen Endabschnitts der ersten Komponente (116) geneigt ist.

4. Erste Komponente (116) nach Anspruch 3, wobei die Führungsformation (118) allgemein kegelstumpfförmig ist und als Trichter für die Zusatzvorrichtung (130) dient.

5. Erste Komponente (116) nach einem der Ansprüche 2 bis 4, wobei die Stege der Führungsformation (118) bogenförmig sind, um die Passage der Zusatzvorrichtung (130) entlang der einwärts weisenden Fläche der Führungsformation (118) zu unterstützen.

6. Erste Komponente (116) nach Anspruch 5, wobei ein Abschnitt der einwärts weisenden Fläche neben der durch die Führungsformation (118) definierten Öffnung (119) konvex ist und ein Abschnitt der einwärts weisenden Fläche neben der Innenfläche der ersten Komponente (116) konkav ist.

7. Erste Komponente (116) nach einem der Ansprüche 2 bis 6, wobei die mehreren Stege voneinander beabstandet sind, so dass Strömungsöffnungen zwischen benachbarten Stegen definiert werden.

8. Erste Komponente (116) nach einem vorherigen Anspruch, wobei die erste Komponente entweder ein Verbinder für eine(n) Beatmungsvorrichtung oder Beatmungsschlauch oder ein(e) Beatmungsvorrichtung oder Beatmungsschlauch mit einem integrierten Verbinder ist.

9. Medizinische Beatmungsvorrichtung, die eine erste Komponente (116) nach einem vorherigen Anspruch und eine zweite Komponente (120) umfasst, wobei der weibliche Endabschnitt der ersten Komponente (116) so ausgelegt ist, dass er einen entsprechenden männlichen Endabschnitt der zweiten Komponente (120) eng aufnimmt, so dass die erste und die zweite Komponente (116, 120) in Verbindung miteinander sind, und wobei der Zugangsanschluss der ersten Komponente (116) das Einführen einer Zusatzvorrichtung (130) ermöglicht, wobei die Führungsformation (118) der ersten Komponente (116) beim Gebrauch die Führungsvorrichtung (130) in die zweite Komponente (120) führt.

10. Medizinische Beatmungsvorrichtung nach Anspruch 9, wobei die Öffnung (119), durch die die Zusatzvorrichtung (130) in die zweite Komponente (120) passieren kann, zentral relativ zur Längsachse des männlichen Endabschnitts der zweiten Komponente (120) positioniert ist.

11. Medizinische Beatmungsvorrichtung nach Anspruch 9 oder Anspruch 10, wobei die medizinische Beatmungsvorrichtung so ausgelegt ist, dass sie einem Patienten Gase zuführt und/oder Gase von einem Patienten empfängt.

## Revendications

1. Premier composant (116) d'un appareil respiratoire médical, le premier composant (116) comprenant une partie d'extrémité femelle adaptée pour recevoir étroitement une partie d'extrémité mâle correspondante d'un second composant (120) de l'appareil respiratoire médical de sorte que les premier et second composants (116, 120) sont en communication l'un avec l'autre, le premier composant (116) étant en outre pourvu d'un orifice d'accès permettant l'insertion d'un dispositif auxiliaire (130), où le premier composant (116) inclut une formation de guidage (118) formée avec une surface de déviation tournée vers l'intérieur qui est disposée pour dévier le dispositif auxiliaire vers l'intérieur loin de la surface intérieure de la partie d'extrémité femelle, en cours d'utilisation lors de l'insertion du dispositif auxiliaire, guidant ainsi le dispositif auxiliaire (130) dans le second composant (120), et empêchant le passage du dispositif auxiliaire dans une partie périphérique de la partie d'extrémité femelle du premier composant dans laquelle le dispositif auxiliaire serait susceptible de se prendre dans, ou d'être obstrué par, un épaulement formé par la partie d'extrémité mâle du second composant, **caractérisé en ce que** la formation de guidage inclut une ou plusieurs ouvertures d'écoulement disposées afin de permettre un écoulement d'air à travers la formation de guidage sans résistance significative de telle sorte que les gaz inhalés et exhalés sont en mesure de s'écouler en cours d'utilisation, mais à travers laquelle le dispositif auxiliaire n'est pas en mesure de passer.

2. Premier composant (116) tel que revendiqué dans la Revendication 1, où la formation de guidage (118) comprend une pluralité de toiles qui s'étendent vers l'intérieur à partir d'une surface intérieure du premier composant (116), et ont des extrémités internes qui définissent une ouverture (119) à travers laquelle le dispositif auxiliaire (130) peut passer.

3. Premier composant (116) tel que revendiqué dans la Revendication 2, où les toiles s'étendent à la fois vers l'intérieur et axialement le long de la partie d'extrémité femelle du premier composant (116) vers la partie d'extrémité mâle du second composant (120) de sorte que la surface tournée vers l'intérieur de la formation de guidage (118) est inclinée par rapport à la surface intérieure de la partie d'extrémité femelle du premier composant (116).

4. Premier composant (116) tel que revendiqué dans la Revendication 3, où la formation de guidage (118) est généralement de forme tronconique, et agit comme un entonnoir pour le dispositif auxiliaire (130).

5. Premier composant (116) tel que revendiqué dans une quelconque des Revendications 2 à 4, où les toiles de la formation de guidage (118) sont de forme arquée de manière à faciliter le passage du dispositif auxiliaire (130) le long de la surface tournée vers l'intérieur de la formation de guidage (118).

6. Premier composant (116) tel que revendiqué dans la Revendication 5, où une partie de la surface tournée vers l'intérieur adjacente à l'ouverture (119) définie par la formation de guidage (118) est de forme convexe, et une partie de la surface tournée vers l'intérieur adjacente à la surface intérieure du premier composant (116) est de forme concave.

7. Premier composant (116) tel que revendiqué dans une quelconque des Revendications 2 à 6, où la pluralité de toiles est espacée de sorte que des ouvertures d'écoulement sont définies entre des toiles adjacentes.

8. Premier composant (116) tel que revendiqué dans une quelconque revendication précédente, où le premier composant est soit un connecteur pour un dispositif respiratoire ou tube respiratoire, soit un dispositif respiratoire ou tube respiratoire ayant un connecteur solidaire.

9. Appareil respiratoire médical comprenant un premier composant (116) tel que revendiqué dans une quelconque revendication précédente et un second composant (120), la partie d'extrémité femelle du premier composant (116) étant adaptée pour recevoir étroitement une partie d'extrémité mâle correspondante du second composant (120) de sorte que les premier et second composants (116, 120) sont en communication l'un avec l'autre, et l'orifice d'accès du premier composant (116) permettant l'insertion d'un dispositif auxiliaire (130), où la formation de guidage (118) du premier mode de réalisation (116) guide le dispositif auxiliaire (130) dans le second composant (120) pendant l'utilisation.

10. Appareil respiratoire médical tel que revendiqué dans la Revendication 9, où l'ouverture (119) à travers laquelle le dispositif auxiliaire (130) peut passer dans le second composant (120) est située de manière centrale par rapport à l'axe longitudinal de la partie d'extrémité mâle du second composant (120).

11. Appareil respiratoire médical tel que revendiqué dans la Revendication 9 ou Revendication 10, où l'appareil respiratoire médical est adapté pour fournir des gaz à un patient et/ou recevoir des gaz d'un patient.
